# EUROPEAN PATENT SPECIFICATION

(11) **EP 1 414 860 B1**
(45) Date of publication and mention of the grant of the patent: **30.12.2009**
(21) Application number: 02727309.3
(22) Date of filing: 25.04.2002
(51) Int. Cl.: C07K 16/18, G01N 33/577, G01N 33/68, G01N 33/72

(54) **ANTI-GLYCATED HEMOGLOBIN PAN-SPECIFIC MONOCLONAL ANTIBODY**
PAN-SPEZIFISCHER MONOKLONALER ANTIKÖRPER DER GEGEN GLYCOSILIERTES HÄMOGLOBIN GERICHTET IST
ANTICORPS MONOCLONAL PAN-SPECIFIQUE ANTI-HEMOGLOBINE GLYCOSYLEE

(30) Priority: 26.04.2001 DK 200100656
(43) Date of publication of application: 06.05.2004
(73) Proprietor: Dako Denmark A/S, 2600 Glostrup (DK)
(72) Inventor: JENSEN, Sussie, Steen, DK-2600 Glostrup (DK); PII, Kurt, DK-2750 Ballerup (DK); SIMONSEN, Anna, Carina, Wiborg, DK-2800 Kgs. Lyngby (DK)
(86) International application number: PCT/DK2002/000267
(87) International publication number: WO 2002/088185

(56) References cited:
- EP-A- 0 315 864
- DE-A- 3 439 610
- US-A- 4 727 036

## Description

This invention relates to the determination of the amount of all structural variants of glycated hemoglobin in human blood samples. The determination of the rate of glycation of hemoglobin in an individual's blood provides a useful index of glucose level control in diabetics.

In particular, the present invention concerns the preparation of monoclonal antibodies or a fragment thereof, which recognize specifically the glycated N-terminal peptide residue in such human hemoglobin.

Patients afflicted with diabetes are incapable of metabolizing glucose in a conventional manner resulting in a build-up of glucose in their blood and urine. Conventionally, the glucose level in such body fluids is taken as a measure of the state of the diabetic condition, which in turn is used as a guide for the amount of insulin or other agent to be taken or of the need to change the patient's diet.

This works moderately well except that the glucose level may fluctuate widely in dependence on time and content of the last meal, the last insulin injection, and the like. Thus, the reading will reflect an instantaneous condition, which may not truly identify the longer-term state of the diabetic condition.

It is known that another effect of the diabetic condition is an increase in the amount of glycated hemoglobin in the blood of the diabetic. Hemoglobin (Hb) is a protein tetramer consisting of four chains (subunits) of amino acids, each of about 143 units and having a total molecular weight of approximately 64,000 g/mol. The N-terminal valine of the β-subunit in hemoglobin can react with glucose. The glycation of hemoglobin occurs by a non-enzymatic reaction involving glucose and the alpha-amino group of valine. Following a Schiff base formation between the reactants, the glucose undergoes an Amadori rearrangement forming 1-deoxyfructo-valine. The final complex binding between 1-deoxyfructose and valine is covalent and irreversible. The glycation reaction is governed by the concentration of the reactants, e.g., hemoglobin and glucose. The Diabetes Control and Complication Trial (DCCT) reference interval for non-diabetic individuals is approximately 4-6% glycated hemoglobin. Hemoglobin tetramers with a 1-deoxyfructo-valine on the N-terminus of a β-chain are identified as being glycated hemoglobin; e.g. Hb1c or HbA1c, HbC1c or HbS1c.

HbC trait has a prevalence of 30% in parts of sub-Saharan and 2.3% among African Americans. HbS trait, the most commonly variant of Hb in US, has a prevalence of 7.8% of African Americans. All together, at least 10% of black Americans have either HbC or HbS trait. The world population is getting more and more mixed with respect to race. Therefore, it is increasingly important to have an antibody that reacts with all structural variants of glycated hemoglobin in the same manner. If the available antibody do not react with all structural variants of glycated hemoglobin, Hb1c percent measurements from patients with traits of HbS and/or HbC will be incorrect.

Glucose levels in diabetics are sufficiently high to increase the rate of glycation depending directly on the glucose level in the blood, which reflects the severity of the diabetic condition. Therefore, the hemoglobin A1c level is raised to about 6 to 12%. Since the circulating life span of hemoglobin A is about 120 days, a glycated hemoglobin measurement will give a value, which reflects an average glucose level for that period. Notably a meal high in glucose will not be reflected in instantly high levels of glycated hemoglobin or serum albumin. Thus, measurement of the glycated-hemoglobin content gives a truer picture of the average circulating glucose levels and thus a truer picture of the long-term condition of the patient.

A common method used to determine the level of glycated hemoglobin involves passing a lysed blood sample through a boronate affinity column. The method determines the total glycated hemoglobin, including HbA_{1c} and ketoamine structures formed on lysine and N-terminal valine residues of both the α- and β-chains of hemoglobin. The column is washed and the glycated hemoglobin determined spectrophotometrically.

Microparticle enhanced turbidimetric immunoassay techniques are available for automatic analyses by instruments. These instruments are not dedicated to a certain diagnostic assay and have a very high sample cycle time. Currently, the utility of this technique for determination of glycated hemoglobin is growing as rutine clinical analysis. Some of these immunoassays are based on the principle of microparticle enhanced competitive turbidimetric inhibition immunoassay techniques.

US Patent no. 4,247,533 discloses an analytical technique
wherein antibodies to HbAlc were reportedly raised in a sheep by injection of HbAlc and absorbed with nonglycated hemoglobin to provide polyclonal antibodies, which distinguished between HbAlc and nonglycated hemoglobin. Such antibodies then form the basis for a test to determine the proportion of glycated hemoglobin in a sample.

In the prior art only the hemoglobin A_{1c} antibody and how the antibody is able to react with the A variant of glycated hemoglobin is described.

US4727036 describes two different monoclonal antibodies specific for two different epitopes within the N-terminus of beta-chain of human hemoglobin, said epitopes comprising the last N-terminal glycosylated amino acid residue of the hemoglobin A_{1c}. DE3439610 describes monoclonal antibodies produced against a glycated four- or five-amino acid long peptide having a sequence corresponding to the sequence of the N-terminal four- or five- amino acid residues fragment of the beta chains of glycated variants of human hemoglobin HbA1c, HbC1c and HbS1c. The antibodies are capable of distinguishing glycosylated hemoglobin from non-glycated, however whether the antibodies bind all structural variant(s) of the glycated hemoglobin is not determined.

The influence of the othe variants of glycated hemoglobin is not mentioned and a monoclonal antibody against all structural variants of glycated-hemoglobin has neither been considered. Due to the fact that the antibody does not recognise HbS1c and HbC1c, immunoassay measurement of glycated-hemoglobin percentages in subjects with HbS1c and HbC1c using that antibody will lead to truly decreased percentages of glycated hemoglobin.

An Hb1c antibody, which is specific for all structural variants of Hb1c, is considered as pan specific.

Accordingly, there remains a need for an antibody of the above-mentioned kind, which does not exhibit the above-identified drawbacks.

It is accordingly an objective of the present invention to provide an antibody, which is pan-specific, i.e. specific for all structural variants of glycated hemoglobin.

It is also the objective to provide an antibody for determining the long-term blood sugar level of a patient based on an immunoassay determination of HbA1c, HbC1c and HbS1c.

It is another objective of the invention to provide such an antibody for determining the content of glycated hemoglobin, HbAlc, HbC1c and HbS1c, in a patient's whole blood sample.

These and the other objects are achieved by the invention as defined in the claims.

The invention relates to a monoclonal antibody or a fragment thereof, which is pan-specific, i.e. able to react with all structural variants of glycated-hemoglobin. The structural variants of glycated-hemoglobin are preferably HbAlc, HbC1c and HbS1c.

The monoclonal antibody preferably leads to improvement of existing immunoassay with respect to measurements of the level of glycated hemoglobin in patients with HbS and HbC.

The monoclonal antibody of the present invention is **characterized in that** the antibody comprises an antibody combining site, which binds specifically to the glycated N-terminal peptide sequence of human hemoglobin A, C and S β-chains.

The monoclonal antibody reagent of the present invention is characterized by its specific binding affinity for the linear epitope in glycated hemoglobin. Therefore, as used herein the term "antibody reagent" or "antibody combining site" will refer to any material however obtained which comprises a monoclonal antibody combining site specific for such peptide epitope. Such term therefore includes whole antibodies as well as appropriate fragments or polyfunctionalized forms thereof. When in the form of whole antibody, it can belong to any of the classes and subclasses of known immunoglobulins, e.g. IgG, IgM and so forth. Any fragment of any such immunoglobulins which retains specific binding affinity for the peptide epitope can also be employed, for instance, the fragments of IgG conventionally known as Fab, Fab' and F(ab')₂. In addition, aggregates, polymers, derivates, conjugates, and hybrids of immunoglobulins, or their fragments can be used where appropriate. Furthermore, single chain antibodies or any other fragment obtained by recombinant means can be used where appropriate.

The glycated residue is the distinguishing structural feature of HbAlc, HbSlc and HbClc. An antibody of the present invention requires an epitope or determinant site comprising minimally the 1-deoxyfructosyl carbohydrate unit, formed upon Amadori rearrangement of the reaction product between glucose and the terminal amine, and a peptide sequence extending therefrom comprising at least one of the amino acid units of the HbAlc, HbSlc and HbClc N-terminal sequence in the position corresponding to the native HbAlc, HbSlc and HbClc sequence. The other amino acid units in the peptide sequence characterizing the epitope may be the same or different as those appearing in the native HbAlc, HbSlc and HbClc sequence. In this way, the epitope is characterized by at least two contact or binding sites with the antibody, which sites are unique to the glycated N-terminal HbAlc, HbSlc and HbClc sequence.

The antibody will preferably specifically bind a glycated peptide residue of the formula:
Glycosyl-(NH)-Val-His- or Glycosyl-(NH)-Val-His-AA,
wherein Glycosyl-(NH)-Val represents a non-enzymatically glycated valine residue, His represents the second amino acid in the native β-chain of hemoglobin and AA means one or more additional amino acid residues.

In a preferred embodiment AA is a sequence of from 1 to 3 amino acids corresponding to a part of the N-terminal β-chain of all structural variants of human hemoglobin. AA is preferably -Leu-Aa; -Leu-Thr-Aa; -Leu-Thr-Pro-Aa, and Aa designates the remaining number of amino acids in the AA group.

A preferred monoclonal antibody or a fragment thereof according to the invention is obtainable using the hybridoma DAK Hb1c-1, which is deposited at Deutsche Sammlung von Mikroorganismen und Zellkulturen GmbH (DSMZ) as accession no. DSM ACC2495.

A method of producing a monoclonal antibody or a fragment thereof according to the invention comprising the step of raising an antibody against an immunogen comprising a glycated peptide chemically linked to an immunogenic carrier material, the glycated peptide having from 2 to 5 amino acid units corresponding to the N-terminal in the β-chain of human hemoglobin. The immunogen is preferably of the formula:

### [Fructosyl-(NH)-Val-His-AA-R]ₙ-Carrier

wherein Fructosyl-(NH)-Val represents a nonenzymatic glycated valine residue, His represents the second amino acid in the native β-chain of hemoglobin, AA means one or more additional amino acid residues, R is a linking group, Carrier is an immunogenic carrier material, and n is an integer from 1 to the number of available coupling sites on said carrier.

In a preferred embodiment AA is one or more additional amino acid residues. In another preferred embodiment AA is a sequence of from 1 to 3 amino acids corresponding to the N-terminal part of the β-chain of all structural variants of human hemoglobin.

Linking group R can be essentially any convenient and stable structure. Such linking group R will usually be in the form of an aliphatic chain comprising between 1 and approximately 20 atoms, excluding hydrogen, and including heteroatoms such as nitrogen, oxygen, and sulfur. The glycated residue can be joined through a variety of groups to form linking chain R, including methylene, ether, thioether, imino, and the like. One skilled in the art will have a wide variety of linking groups from which to choose to prepare the immunogen.

In a preferred embodiment the carrier is an immunogenic protein or peptide other than human hemoglobin.

The immunogen used to stimulate production of appropriate immunoglobulins in the most general sense will comprise one or more of the glycated peptide residues chemically linked to an immunogenic carrier material. The immunogenic carrier material can be selected from any of those conventionally known having functional groups available for coupling to the glycated peptide residue. In most cases, the carrier is a protein or polypeptide, although other materials such as carbohydrates, polysaccharides, lipopolysaccharides, nucleic acids, and the like of sufficient size and immunogenicity can likewise be used. For the most part, immunogenic proteins and polypeptides will have molecular weights between 4,000 g/mol and 10,000,000 g/mol, preferably greater than 15,000 g/mol, and more usually greater than 50,000 g/mol. Generally, proteins taken from one animal species will be immunogenic when introduced into the blood stream of another species. Particularly useful proteins are albumins, globulins, enzymes, hemocyanins, glutelins, proteins having significant nonproteinaceous constituents, and the like.

In a preferred embodiment the glycated N-terminal peptide residue of the native HbAlc, HbSlc and HbClc molecule is made accessible to the monoclonal antibody or a fragment thereof of the present invention by appropriate denaturation or digestion of the protein in the sample to be assayed.

In another preferred embodiment the glycated fragment can be produced by chemical or enzymatic digestion of naturally occurring glycated hemoglobin, e.g. HbAlc, HbSlc and HbClc. This fragment can be coupled to a carrier using classical coupling procedures, e.g., glutaraldehyde or carbodiimide, and the conjugate used as an immunogen.

The antibody or a fragment thereof according to the invention may preferably be produced by the following step:

### A. Preparation of peptide immunogen

To obtain antibodies against the glycation site of N-terminal valine residue on the β-chain of Hb A, S and C a short peptide (pentamer) is preferably used as an immunogen. The antigen may preferably be Hb1c 4-mer plus cysteine, i.e. fructosyl-V-H-L-T-C.

### B. Immunization of mize with human Hb1c peptides

The general method used to develop hybridomas secreting monoclonal antibodies is well known to a person skilled in the art.

An illustration of the techniques utilized in the practice is described in Journal of Immunological Methods, 1980, 39:285.

Female F1 hybrids of CF1 x BALB/c mice may preferably be used, but mice of other strains may also be used. The immunization plan and the concentration of peptide-carrier complex may be selected to form a sufficient amount of antigenically stimulated lymphocytes. In a preferred embodiment mice are immunized intraperitoneally with 50 micrograms of purified protein derivates (PPD, a partly unknown mixture of denatured proteins from *M. tuberculosis*) in complex with the Hb1c peptide four times at 2-week intervals. 3-5 days after the final immunization, the spleen cells are extracted from the animal for fusion.

Monoclonal antibodies produced by hybridomas from other species may also preferably be used.

### C. Cell fusion

The spleen is aseptically taken out from the immunized mouse, and a suspension of the spleen cells is prepared. The spleen cells are fused with mouse myeloma cells from a suitable cell line in the presence of a suitable fusion promotor. In a preferred embodiment the preferred ratio of the spleen cells to the myeloma cells is from about 20:1 to about 2:1, and a fusion medium is suitably used in an amount of 0.5 to 1.5 ml per about 10⁸ spleen cells.

The mouse myeloma cells used for cell fusion are well known. P3-X63-Ag 8.653 may preferably be used as a mouse myeloma cell.

The fusion promoter is preferably polyethylene glycol having an average molecular weight of 1000 to 4000 g/mol. Other fusion promoters known in the art can also be used. In a preferred embodiment polyethylene glycol having an average molecular weight of 1500 may be used.

### D. Screening of the fused cells

In a separate container (such as a microtiter plate), a mixture composed of the unfused spleen cells, unfused mouse myeloma cells and the fused hybridoma cells is preferably diluted with a selective medium not supporting the unfused mouse myeloma cells and cultured for a period sufficient to kill the unfused mouse myeloma cells (about 1 week). A culture medium having drug resistance (for example, resistance to 8-azaguanine) and not supporting the unfused mouse myeloma cells, such as HAT medium, is used. In the selective medium, the unfused myeloma cells die. Since the unfused spleen cells are untransformed cells, they die away after a certain period of time (1 week). The fused cells, on the other hand, can survive in the selective medium since they have both the tumoral nature of the parent myeloma cells and the nature of the parent spleen cells.

### E. Determination of glycated hemoglobin by ELISA

The determination is preferably based on the principle of specific immunologic recognition and reaction between a monoclonal antibody and the antigenic epitope to which the antibody uniquely and specifically binds. The recognition and binding may be detected, for example, by an ELISA test, wherein blood samples or calibrators are immobilized on a solid phase support, such as the bottom of a plastic well. By immobilizing human hemoglobin the antigenic epitope may be exposed for antibody binding. The antibody, secondary enzyme-labeled antibody and enzyme substrate may interact with the immobilized antigen (human hemoglobin) thereby antibody-antigen complexes are preferably formed. A number of dilution, incubation and washing steps, then allow separation of bound and free reagents. A colour forming reaction takes place as a result of binding of the antibody to antigen and the consequent reaction of the enzyme upon its substrate interaction. The formation of colour indicates the presence of Hb1c in the blood sample, and the intensity of the colour provides a quantitative measure of the amount of glycated epitope in the sample. The antibody of the present invention may be used to measure glycated hemoglobin in other immunological assay formats known in the art.

### F. Cloning of the hybridoma cells producing the desired antibody and the production of the antibody

Hybridoma cells capable of producing the desired antibody are cloned by a suitable method such as limiting dilution method, and the desired antibody can be produced by the following method. Hybridoma cells are cultured in a suitable medium for a certain period of time, and the monoclonal antibody produced by the hybridoma cells can be isolated from the supernatant of the culture.

The invention also relates to a method of using a monoclonal antibody or a fragment thereof.

The antibodies or a fragment thereof according to the invention can preferably be used in conventional manner to react with blood samples containing unknown quantities of glycated hemoglobin and the extent of reaction can be compared with calibrated standards to determine the extent of glycation. The read-out can be by fluorescence, by immunoassay, or the like, by joining suitably readable groups to the monoclonal antibodies in known manner without loss of their binding power for the glycated epitope in HbAlc, HbSlc and HbClc.

The monoclonal antibodies of the present invention are specific for binding to all structural variants of glycated N-terminal peptide residue preferable found in HbA, HbS and HbC. The antibodies are able to bind to the epitope in the native HbAlc, HbSlc and HbClc molecule when the epitope is appropriately exposed. Steric access to the epitope can be obtained in any effective manner. Exposure of the epitope in the intact protein is understood to be accomplished by a physical or chemical denaturation or digestion at least in the region of the epitope. Such denaturation or digestion can be localized to the region of the epitope or can involve a more general, or even substantially complete denaturation of the tertiary, and additionally the secondary structure of the protein, or partial or complete digestion of the protein.

In a preferred embodiment the antibody binds specifically to the glycated N-terminal peptide sequence in the β-chain of human hemoglobin A1c, C1c and S1c after having been exposed sufficiently to provide steric access thereto.

Denaturation can be accomplished in a variety of ways including conventional treatment of the protein by physical means such as heat, sonication, high or low pH and, as is preferable, chemical denaturation by interaction with an agent or chaotrope in solution.

Protein denaturation can be most effectively accomplished if combinations of chemical and/or chemical and physical means are used, e.g. guanidine and heat, guanidine and SDS, or guanidine and dithiothreitol. Particularly strong chaotropes such as guanidine are preferred. Of course, denaturing conditions which result in substantial insolubilization, aggregation, or precipitation of the protein implying an insignificant amount of the exposed epitope is accessible to the solution for antibody binding will be avoided. A sufficient amount of the denatured protein must remain in solution or suspension in order to obtain useful immunobinding. The extent of solubilization necessary will depend upon the circumstances of the intended or desired binding.

In a preferred embodiment the glycated peptide sequence is exposed to the antibodybinding site by physical or chemical denaturation or digestion.

In another preferred embodiment the glycated peptide sequence is exposed to the antibody binding site by denaturation with a chaotropic agent. The chaotropic agents include, without limitation, guanidine, urea, and various detergents such as sodium dodecylsulfate (SDS) and others, without limitation, including deoxycholoate and certain bile salts, 3-(3-cholamidopropyl)-dimethyl-ammonio-1-propanesulfonate, organic solvents such as methanol, propanol, acetonitrile and certain salts such as potassium thiocyanate. Non-ionic detergents such as Triton X^{®}, Tween^{®}, nonidet NP-40^{®}, and octyl-glucosides can also function as protein denaturants.

A significant amount of HbAlc, HbSlc and HbClc in a particular blood sample can be denatured to expose the glycated epitope for antibody binding by combining the sample, e.g., whole blood or red cell hemolysate, with an aqueous solution of the chaotrope present at sufficient concentration to denature any HbAlc, HbSlc and HbClc in the resulting aqueous mixture. Where whole blood is the sample, the chaotrope also serves to lyse red blood cells to release hemoglobin and to inactivate proteases.

The denaturation process may be significantly accelerated by heating the mixture for a short period of time. It has been found that at temperatures below 37°C , denaturation by the chaotrope can take from one to several hours, whereas at temperatures above 50°C sufficient denaturation can be attained in a minute or less. In order to prevent significant denaturation of the antibody and other proteinaceous reagents to be subsequently added to the mixture, the sample-chaotrope mixture will normally be diluted as a separate step or by addition of reagent solutions to a level that the chaotrope is substantially ineffective to denature such reagents, yet will preserve the exposure of the epitope by preventing significant renaturation of HbAlc, HbSlc and HbClc.

In a preferred embodiment the antibody binds specifically to a glycated N-terminal peptide sequence in the β-chain of glycated human hemoglobin, which has been adsorbed to a solid phase, wherein the solid phase preferably is selected from the group consisting of polystyrene, divinylbenzene, butadiene, polycarbonate, polyacrylamide, polyacrylic acid, polyacryl amide, polyethylene, polypropylene, fluorinated polymers, poly amides, or co-block polymers hereof, gold, carbon, cellulose, derivatized cellulose, or glass.

In a preferred embodiment the monoclonal antibody or a fragment thereof may be used for the determination of the level of glycated hemoglobin in human blood. The monoclonal antibody or a fragment thereof may also preferably be used for the determination of the level of glycated HbS and/or HbC in human blood.

In a further preferred embodiment the monoclonal antibody or a fragment thereof may be used for the determination of the level of glycated heterozygous HbA, HbS and/or HbC in human blood.

In a further preferred embodiment there is provided a turbidimetric two-microsphere method for determination of the level of glycated hemoglobin. This method comprises mixing a sample with microspheres and microspheres on which the monoclonal antibody or a fragment thereof is bound; the latter is referred to as immunoparticles, or with microspheres and subsequently with immunomicrospheres. The resulting immunoagglutinates is measured turbidimetrically.

The microspheres are preferably selected from the group consisting of polystyrene, divinylbenzene, butadiene, polycarbonate, polyacrylamide, polyacrylic acid, polyacryl amide, polyethylene, polypropylene, fluorinated polymers, poly amides, or co-block polymers hereof, gold, or silica.

Microspheres to be used in the method of the present example have a particle size of from 0.01 to 5 micro meters, which are used by dispersing in a usually used buffer solution.

The following experimental non-limiting examples are intended to illustrate certain features and embodiments of the invention.

### Example 1:

The 1-deoxyfructosyl-Val-His-AA peptide was synthesized using the solid-phase method of Sheppard and Atherton as described in international patent application no. WO 86/03494. The peptide was synthesized with fluorenylmethoxycarbonyl (Fmoc) amino acids, using PyBOP (benzotriazole-1-yl-oxy-tris-pyrrolidino-phosphorium hexafluorophosphate) and HOBt (N-hydroxy-benzotriazole) as coupling reagents.

Cysteine and histidin were used with trityl (trt), and threonin with tertiary butyl (tBu) side-chain protection. Leucin was used without side-chain protection.

The fructose amino acid conjugate, N-(deoxy-D-fructos-1-yl)-L-valine, was synthesized by modifications of known methods. In short, Di-isopropyliden protected glucose was activated as the trifluate and conjugated to the benzyl ester of valine. The resulting 2,3:4,5-Di-*O-*isopropyliden-N-(deoxy-D-fructos-1-yl)-L-valine-benzyl ester was hydrogenated over Pd/C in ethanol, lyophilized, and finally used in the peptide synthesis without Fmoc protection. The purity of the product was verified by TLC analysis and ¹H or ¹³C NMR spectra.

The peptide was synthesized by automated solid phase synthesis (Crystal instrument from Calbiochem-Novabiochem Ltd) cleaved from the resin with TFA (trifluoroacetic acid) - TES (triethylsilan) - Glycerol - H₂O (90% : 4% : 3% : 3%), precipitated and washed with methyl-tertiarybutyl-ether, centrifuged and lyophilised.

Purification was performed by HPLC (C₁₈ reversed-phase column, using a step-wise gradient of typically 5-25% MeCN in 0.1% TFA). The identity of the peptide was verified by MALDI-TOF mass spectroscopy and the purity was finally assured by an analytical HPLC analysis to be better than 90%.

### Example 2:

Four weeks before first immunization Bacille Calmette Guérin (BCG) vaccine was injected to prime the immune response in the mice as described in Giba Foundation Symposium, 1986, 119: 25.

To make the peptide immunogen it was conjugated to a carrier mixture, Tuberculin PPD. Peptide and carrier (PPD) were conjugated with glutardialdehyde, i.e. CHO-(CH2)₃-CHO. Glutardialdehyde reacts with amino-groups on the peptide and the carrier.

### The immunogens thus have the following formula:

Fructosyl-Val-His-Leu-Thr-Cys-CH-(CH2)₃-CH-PPD

### Example 3:

Female F1 hybrids of CF1 x BALB/c mice (12 weeks old) primed with BCG-vaccine were immunized with the peptide-PPD complex described above. Mice were immunized four times intraperitoneally with a mixture of 50 micrograms of peptide-PPD complex dissolved in phosphate buffered saline (PBS) and aluminium hydroxide (1 mg/dose). A mouse with high titer of antibodies against glycated hemoglobin was selected for fusion. Three days after the final immunization, spleen cells from the immunized mouse was used for cell fusion.

Spleen cells from the selected immunized mouse and myeloma cells of BALB/c origin (P3-X63-Ag 8.653) were mixed in a ratio of about 3:1 and fused in the presence of 50% polyethylene glycol 1500 (a product of Roche Diagnostics GmbH)(J. W. Goding: Monoclonal Antibodies: Principle and Practice, Academic Press, San Diego, 1996). The fused cells were suspended in RPMI-1640 medium, containing 10% FCS, hypoxanthine, aminopterin and thymidine (HAT media) to give a concentration of 1×10⁶ cells/mL. The suspension was distributed to 96-well microplates (Costar) in an amount of 200 µL per well.

The cells were incubated in a CO2 incubator (5% CO2, 37°C). After 14 days hybridoma cells were screened.

Supernatants containing antibodies produced by the hybridoma cell cultures were detected by an ELISA technique using microtiter plates coated with hemoglobin calibrator containing 4.7% HbAlc and 16.7% HbA1c as antigens. 120 µg/mL of the calibrators were immobilized onto plastic microtiter wells using a carbonate/bicarbonate buffer (pH 9.6) for 15 minutes at 37°C. After a washing step that removes unbound antigen the hybridoma culture supernatants diluted in 50 mM Tris-HCl buffer containing 0.1% Tween 20 were added to each well and allowed to react for one hour at room temperature. After another washing step to remove unbound antibody a conjugate of a goat anti-mouse antibody and the enzyme horseradish peroxidase was added. After incubation and a third washing step the bound conjugate was detected by reaction with the substrate 3,3,5,5-tetramethylbenzidine (TMB). The reaction was stopped with sulphuric acid and the absorbance at 450 nm was measured. Only cells from positive wells reacting with glycated hemoglobin calibrators, 4.7% HbAlc and 16.7% HbA1c, with increasing intensity were selected.

These positive cells were cloned three times by limiting dilution method. The resulting clone, DAK Hb1c-1, was suspended in 90% FCS; 10% DMSO and stored at -150°C.

### Example 4:

A linear antibody epitope mapping is the systematic screening of all possible peptides derived from a polypeptide or protein sequence. Epitope mapping yields information on the linear stretch of amino acids and derivatives that form an interaction site to a given antibody.

The specificity of DAK Hblc-1 for the glycated epitope of hemoglobin β-chain has been determined.

A peptide library containing 1-5 amino acid long peptides manually synthesized from the C- to the N-terminal end on a solid phase (Abimed membrane) using Fmoc as protecting group of the N-terminal end of amino acids. Detailed description of peptide synthesis is described in Example 1. The following four peptides were synthesized:
Fructosyl-Hb β-chain
   1-5 amino acid long peptide analogs of N-terminal 1-deoxyfructosyl-hemoglobin β-chain.
Hb β-chain
   1-5 amino acid long peptide analog of N-terminal hemoglobin β-chain (minus 1-deoxyfructosyl β-chain; backbone control).
Hb α-chain
   5 amino acid long peptide analog of N-terminal hemogobin α-chain (negative sequence control).
Fructosyl-Hb α-chain
   5 amino acid long peptide analogs of hemoglobin α-chain incl. 1-deoxyfructosyl at the N-terminal valine residue (negative 1-deoxyfructosyl sequence control).

The membranes with peptide spots were incubated with DAK Hb1c-1 and an irrelevant antibody (a negative antibody control, anti-troponin I, DAKO, product no. O 9528). Thereafter, the membranes were incubated with HRP conjugated goat-anti-mouse antibodies (DAKO, product no. P 0447) and visualized using 3-amino-9-ethylcarbazole as substrate.

| ***Number* of Amino** ***Acids*** | 5 | 4 | 3 | 2 | 1 |
|---|---|---|---|---|---|
| ***Peptide name*** | | | | | |
| Fructosyl-Hb β-chain | ++ | ++ | ++ | + | - |
| Hb β-chain | - | - | - | - | - |
| Hb α-chain | - | *N.D.* | *N.D.* | *N.D.* | *N.D.* |
| Fructosyl-Hb α-chain | - | *N.D.* | *N.D.* | *N.D.* | *N.D.* |

Table 1: Spot peptide library for epitope mapping of anti-Hb1c antibody clone DAK Hb1c-1. Intensity of peptide spots after incubation with anti-Hb1c antibody clone DAK Hb1c-1 followed by visualization are shown; strong intensity (++), medium intensity (+), negative (-), not determined (N.D.).

DAK Hblc-1 reacts moderately with 1-deoxyfructosyl 2-mer of hemoglobin β-chain, strongly with 1-deoxyfructosyl 3-mer to 5-mer hemoglobin β-chain (see Table 1). DAK Hblc-1 does not react with 1-deoxyfructosyl-valine alone. DAK Hblc-1 does not react with non-fructosylated peptides of hemoglobin β-chain, 1-deoxyfructosyl 5-mer hemoglobin α-chain and 5-mer hemoglobin α-chain. In contrast to DAK Hblc-1, the negative antibody control did not react with any of the peptides.

In summary, the specificity of anti-Hb1c antibody clone DAK Hb1c-1 for the 1-deoxyfructosylated N-terminal part of hemoglobin β-chain has been demonstrated.

### Example 5:

Peptides, proteins from a whole blood sample, red cellhemolysate or calibrators were allowed to bind to the surface of a 96-well microtiter plate. Following a washing step to remove unbound proteins, the monoclonal antibody, DAK Hb1c-1, was added. After incubation, the unbound antibody was washed away, and a conjugate of a goat-anti-mouse antibody and the enzyme horseradish peroxidase was added. After another washing step the bound conjugate was detected by reaction with 3,3,5,5-tetramethylbenzidine (TMB) substrate. The reaction was stopped by adding acid to give a colorimetric end point that was read spectrophotometrically.

### Example 6:

HbAo is identical to HbA1c except that there is no 1-deoxyfructosyl linked to the amino-terminus of the hemoglobin β-chain.

The reaction of DAK Hblc-1 with HbAo and 17-mer peptides of HbC₀ and HbS₀ was analysed by the ELISA described above.

When DAK Hblc-1 was added to microtiter plates coated with either HbAo or 17-mers peptides of HbC₀ or HbS₀ the values measured were no higher than the background values.

In conclusion, no reactivity of DAK Hb1c-1 with HbAo, HbC₀ or HbS₀ was demonstrated.

### Example 7:

The reaction of DAK Hb1c-1 with native HbA1c in solution was analyzed in a competitive ELISA. HbA_{1c} calibrator in solution was pre-incubated with DAK Hblc-1 antibody to test if any antigen-antibody complexes were formed. The solution of antigen-antibody was then transferred to microtiter-wells, where the same antigen was adsorbed on the solid phase (immobilized antigen). The antigen in solution was in excess. The antibody not in complex with the antigen in solution was able to bind to the immobilized antigen. A secondary antibody as described above detected the bound DAK Hb1c-1 antibody. As a control the same amount of antibody not pre-incubated with HbA1c calibrator in solution was added to one lane of the microtiter plate and followingly subjected to the protocol described above.

No effect of pre-incubation of DAK Hblc-1 antibody with HbA1c in solution was observed, concluding that DAK Hb1c-1 does not bind to native HbA1c in solution.

### Example 8:

The reactivity of DAK Hblc-1 and HEM13 with human blood containing structural variants of hemoglobin have been analysed by isoelectric focusing (IEF) followed by immunoblotting.

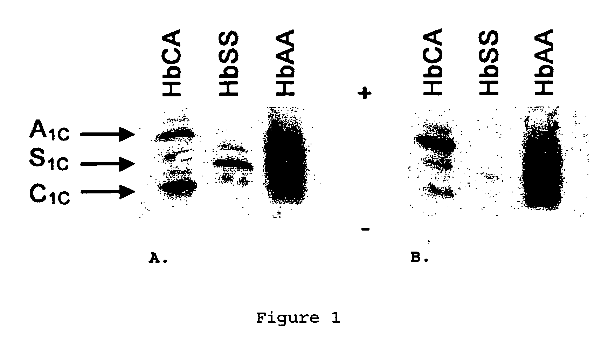

Red cell hemolysate containing HbCA, HbSS and HbAA were electrophoretically separated by Pharmacia's PhastSystem using IEF polyacrylamide gel (PhastGel IEF - 5-8, Pharmacia) in order to separate structural variants of hemoglobin. Following, the gels were subjected to press blotting of proteins onto a PVDF membrane and blocked for non-specific staining with a blocking agent. The membranes were then incubated with DAK Hblc-1 (see figure 1A) and HEM13 (see figure 1B), respectively. HRP-conjugated goat anti-mouse immunoglobulin (DAKO A/S, product no. P 0447) was used as secondary antibody. Finally, HRP activity were visualized using DAKO liquid DAB+ Substrate-Chromogen System (DAKO A/S, product no. K 3468).

A distinct HbS_{1c} band with strong intensity was identified analysing red cell hemolysate from a non-diabetic patient with homozygous HbSS with the monoclonal antibody, clone DAK Hblc-1 (see Figure 1, A). In contrast, weak HbS_{1c} staining was revealed with HEM13 analyzing red cell hemolysate from a patient with homozygous HbSS (see Figure 1B).

Analyzing red cell hemolysate from a non-diabetic individual with heterozygous hemoglobin CA, HbC_{1c} bands with strong and moderate intensity were visualized with DAK Hb1c and HEM 13, respectively. A strong HbA_{1c} band with approximately the same intensity was observed for both DAK Hb1c and HEM13.

As a control a pool of red cell hemolysate from diabetes patients with hemoglobin A was also tested. Other variants of hemoglobin not related to C and S may be a part of this patient pool. Each membrane was incubated with substrate until HbA1c bands with approximately the same intensity were revealed on both DAK Hblc-1 and HEM 13.

Conclusively, we have demonstrated that our pan-specific monoclonal glycated hemoglobin antibody, clone DAK Hb1c-1, reacts with HbA_{1c}, HbS_{1c} and HbC_{1c} from human blood samples.

### Example 9:

Adult hemoglobin is a tetrameric protein, consisting of two α-subunits and two β-subunits, α2β2. Structural variants of Hb contain genetically determined changes in the primary structure of the peptides. A, S and C differ only in the sixth amino acid residue from the N-terminal of the β-chain:
HbA1c fructosyl-V-H-L-T-P-E-E-K-T-A-V-N-A-L-W-G-K.....
HbC1c fructosyl-V-H-L-T-P-K-E-K-T-A-V-N-A-L-W-G-K.....
HbS1c fructosyl-V-H-L-T-P-V-E-K-T-A-V-N-A-L-W-G-K.....

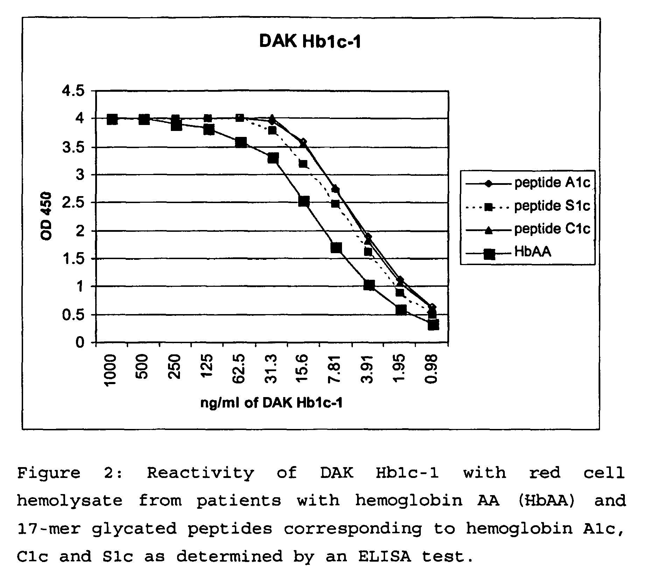

To test if the antibody is pan-specific, i.e. reacting with all structural variants of glycated hemoglobin, we have tested the reaction of DAK Hb1c-1 antibody with three 17-mer glycated peptides corresponding to the N-terminal end of glycated hemoglobin A, C and S β-subunits by ELISA (Figure 2). The peptide sequences are shown above. As a control a pool of red cell hemolysate from diabetes patients with hemoglobin A was also tested by DAK Hblc-1 ELISA.

Monoclonal HbA1c antibody clone HEM13 has been raised by immunization of mice with purified hemoglobin A1c. We have compared the reaction of DAK Hblc-1 and HbA1c antibody clone HEM13 with the glycated hemoglobin peptides and erythrocyte extract of hemoglobin A.

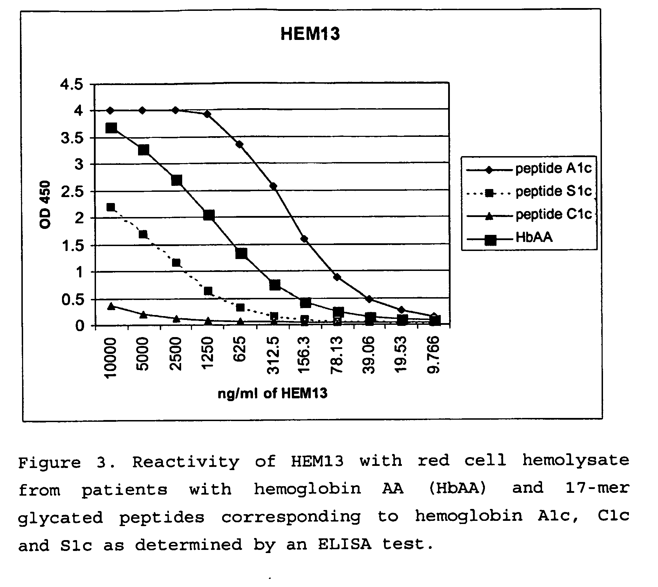

The affinity of DAK Hb1c-1 for the glycated hemoglobin peptides is significantly stronger than the affinity of HEM13 for the same peptides as judged by comparing the titration curves (see Figure 2 and Figure 3).

HEM13 shows different reactions with the A1c, S1c and C1c peptides (Figure 3) whereas DAK Hb1c-1 reacts in the same manner with all three peptides (Figure 1). These results demonstrates that the DAK Hblc-1 antibody is pan-specific.

This example verifies that an antibody that reacts in the same manner with all structural variants of glycated hemoglobin can be raised by injection of a glycated 5-mer peptide corresponding to the first 4 amino acids of all structural variants of glycated hemoglobin A, C and S β-chain plus one irrelevant amino acid (here cysteine).

### Example 10:

Blood samples from the "ERL Educational Programme" have been tested. The HbA1c-percentage of each sample has been determined by cation-exchange chromatography (IFCC/DCCT-values in table 2; IFCC: International Federation of Clinical Chemistry; DCCT: Diabetes Control and Complication Trial). A homozygotic blood sample consists of two identical β-subunits, e.g. AA or SS, whereas heterozygotic samples consists of two different hemoglobin β-subunits, e.g. AS or AC.

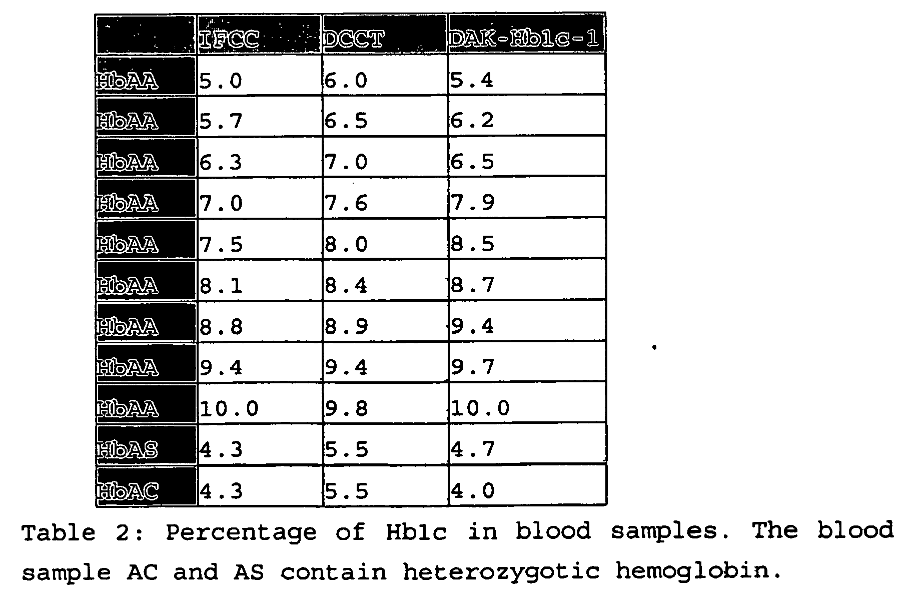

At a range from 4-10% the HbAlc percentage was measured in whole blood samples using DAK Hb1c-1 in the above-mentioned ELISA format. HbAlc percentages at the same levels as the values determined by reference methods recommended by IFCC and DCCT were obtained.

In summary, it is demonstrated that the antibody in question has the correct specificity against HbA1c, HbC1c and HbS1c.

### Example 11:

The level of glycated hemoglobin was measured in a two-microsphere turbidimetry assay. In this set-up glycated hemoglobin was non-covalently adsorbed to microspheres followed by detection of the antigen with glycated hemoglobin antibodies covalently bound to microspheres. The resulting network constituting antigen microspheres complexed with immunomicrospheres were measured turbidimetrically.

## Claims

1. A monoclonal antibody obtainable by hybridoma DAK Hb1c-1 deposited at Deutsche Sammlung von Mikroorganismen und Zellkulturen GmbH (DSMZ) as accession no. DSM ACC2495, or a fragment thereof, wherein said antibody or said fragment is capable of specifically binding HbA1c, HbC1c and HbS1c structural variants of glycated human hemoglobin with a substantially similar affinity.

2. The monoclonal antibody or the fragment thereof according to claim 1, wherein the antibody or the fragment thereof capable of specifically binding to a glycated peptide residue of the formula:
Glycosyl-(NH)-Val-His- or Glycosyl-(NH)-Val-His-AA,
wherein Glycosyl-(NH)-Val represents a non-enzymatically glycated valine residue, His represents the second amino acid in the native β-chain of hemoglobin and AA means 1 to 3 amino acid residues.

3. Use of a monoclonal antibody or a fragment thereof according to claims 1 or 2 for determining the level of glycated human hemoglobin in a sample of human blood in vitro.

4. The use according to claim 3, for determining the level of glycated HbS and/or HbC.

5. The use according to claim 4, for determining the level of glycated heterozygous HbA, HbS and/or HbC.

6. The use according to claims 3, 4 or 5, wherein the level of the glycated hemoglobin in the blood sample is determined in a two-particle turbidimetric assay.

7. The use according to claim 6, wherein the determining comprises a step of mixing the blood sample with
(i) a first population of microsphere particles which comprises particles that are not bound to the monoclonal antibody or the fragment thereof according to claim 1 or 2, and simultaneously with
(ii) a second population of microsphere particles which comprises particles that are bound to the monoclonal antibody or the fragment thereof according to claim 1 or 2.

8. The use according to claim 6, wherein the determining comprises a step of mixing the blood sample with
(i) a first population of microsphere particles which comprises particles that are not bound to the monoclonal antibody or the fragment thereof according to claims 1 or 2, and subsequently with
(ii) a second population of microsphere particles which comprises particles that are bound to the monoclonal antibody or the fragment thereof according to claims 1 or 2.

9. A method for in vitro determining the level of glycated human hemoglobin in a sample, wherein the determining is based on assessment of the level of glycated HbA1c, HbC1c and HbS1c, said method comprising a step of using an antibody according to claims 1 or 2.

10. The method according to claim 9, wherein the sample comprises heterozygous glycated HbA, HbS and/or HbC.

11. The method according to claim 9, wherein said method is for determining the level of glycated HbS and/or HbC.

## Patentansprüche

1. Monoklonaler Antikörper, erhältlich durch das Hybridom DAK Hb1c-1, das bei der Deutschen Sammlung von Mikroorganismen und Zellkulturen GmbH (DSMZ) unter der Hinterlegungs-Nr. DSM ACC2495 hinterlegt ist, oder Fragment davon, wobei der Antikörper oder das Fragment fähig ist, die Strukturvarianten HbA1c, HbC1c und HbS1c des glycierten menschlichen Hämoglobins mit einer im Wesentlichen ähnlichen Affinität spezifisch zu binden.

2. Monoklonaler Antikörper oder Fragment davon gemäß Anspruch 1, wobei der Antikörper oder das Fragment davon fähig ist, einen glycierten Peptidrest der Formel:
Glycosyl-(NH)-Val-His- oder Glycosyl-(NH)-Val-His-AA
spezifisch zu binden, wobei -Glycosyl-(NH)-Val einen nicht enzymatisch glycierten Valinrest darstellt, His die zweite Aminosäure in der nativen β-Kette von Hämoglobin darstellt und AA 1 bis 3 Aminosäurereste bedeutet.

3. Verwendung eines monoklonalen Antikörpers oder eines Fragmentes davon gemäß den Ansprüchen 1 oder 2 zur Bestimmung des Spiegels an glyciertem menschlichem Hämoglobin in einer Probe menschlichen Blutes in vitro.

4. Verwendung gemäß Anspruch 3 zur Bestimmung des Spiegels an glyciertem HbS und/oder HbC.

5. Verwendung gemäß Anspruch 4 zur Bestimmung des Spiegels an glyciertem heterozygotem HbA, HbS und/oder HbC.

6. Verwendung gemäß den Ansprüchen 3, 4 oder 5, wobei der Spiegel an glyciertem Hämoglobin in der Blutprobe in einem turbidimetrischen Zwei-Teilchen-Assay bestimmt wird.

7. Verwendung gemäß Anspruch 6, wobei die Bestimmung einen Schritt des Mischens der Blutprobe mit
i) einer ersten Population Mikrokügelchenteilchen, die Teilchen umfasst, welche nicht an den monoklonalen Antikörper oder das Fragment davon gemäß Anspruch 1 oder 2 gebunden sind, und gleichzeitig mit
ii) einer zweiten Population Mikrokügelchenteilchen, die Teilchen umfasst, welche an den monoklonalen Antikörper oder das Fragment davon gemäß Anspruch 1 oder 2 gebunden sind,
umfasst.

8. Verwendung gemäß Anspruch 6, wobei die Bestimmung einen Schritt des Mischens der Blutprobe mit
i) einer ersten Population Mikrokügelchenteilchen, die Teilchen umfasst, welche nicht an den monoklonalen Antikörper oder das Fragment davon gemäß Anspruch 1 oder 2 gebunden sind, und anschließend mit
ii) einer zweiten Population Mikrokügelchenteilchen, die Teilchen umfasst, welche an den monoklonalen Antikörper oder das Fragment davon gemäß Anspruch 1 oder 2 gebunden sind, umfasst.

9. Verfahren zur in-vitro-Bestimmung des Spiegels an glyciertem menschlichem Hämoglobin in einer Probe, wobei die Bestimmung auf einer Abschätzung des Spiegels an glyciertem HbA1c, HbC1c und HbS1c beruht, wobei das Verfahren einen Schritt umfasst, einen Antikörper gemäß den Ansprüchen 1 oder 2 zu verwenden.

10. Verfahren gemäß Anspruch 9, wobei die Probe heterozygotes glyciertes HbA, HbS und/oder HbC umfasst.

11. Verfahren gemäß Anspruch 9, wobei das Verfahren zur Bestimmung des Spiegels an glyciertem HbS und/oder HbC dient.

## Revendications

1. Anticorps monoclonal pouvant être obtenu par le biais d'un hybridome DAK Hb1c-1 déposé auprès de la Deutsche Sammlung von Mikroorganismen und Zellkulturen GmBH (DSMZ) sous le n° d'accession DSM ACC2495, ou fragment de celui-ci, où ledit anticorps ou ledit fragment est capable de se lier spécifiquement à des variantes structurelles HbC1c, HbC1c et HbS1c d'hémoglobine humaine glycosylée ayant une affinité pratiquement similaire.

2. Anticorps monoclonal ou fragment de celui-ci selon la revendication 1, où l'anticorps ou le fragment de celui-ci est capable de se lier spécifiquement à un résidu peptidique glycosylé ayant pour formule :
Glycosyl-(NH)-Val-His- ou Glycosyl-(NH)-Val-His-AA,
où Glycosyl-(NH)-Val- représente un résidu de valine glycosylé de manière non enzymatique, His représente le deuxième acide aminé dans la β-chaîne native d'hémoglobine et AA signifie de 1 à 3 résidus d'acides aminés.

3. Utilisation d'un anticorps monoclonal ou d'un fragment de celui-ci selon la revendication 1 ou 2, destiné à déterminer le niveau d'hémoglobine humaine glycosylée dans un prélèvement sanguin humain in vitro.

4. Utilisation selon la revendication 3, destinée à déterminer le niveau de HbS et/ou de HbC glycosylées.

5. Utilisation selon la revendication 4, destinée à déterminer le niveau de HbA, HbS et/ou de HbC hétérozygotes glycosylées.

6. Utilisation selon la revendication 3, 4 ou 5, dans laquelle le niveau de l'hémoglobine glycosylée dans le prélèvement sanguin est déterminé lors d'un essai turbidimétrique à deux particules.

7. Utilisation selon la revendication 6, dans laquelle la détermination comprend une étape consistant à mélanger le prélèvement sanguin avec
(i) une première population de particules microsphériques qui comprend des particules qui ne sont pas liées à l'anticorps monoclonal ou au fragment de celui-ci selon la revendication 1 ou 2, et simultanément avec
(ii) une deuxième population de particules microsphériques qui comprend des particules qui sont liées à l'anticorps monoclonal ou au fragment de celui-ci selon la revendication 1 ou 2.

8. Utilisation selon la revendication 6, dans laquelle la détermination comprend l'étape consistant à mélanger un prélèvement sanguin avec
(i) une première population de particules microsphériques qui comprend des particules qui ne sont pas liées à l'anticorps monoclonal ou au fragment de celui-ci selon la revendication 1 ou 2, et ensuite avec
(ii) une deuxième population de particules microsphériques qui comprend des particules qui sont liées à l'anticorps monoclonal ou au fragment de celui-ci selon la revendication 1 ou 2.

9. Procédé de détermination in vitro du niveau d'hémoglobine humaine glycosylée dans un prélèvement, où la détermination est basée sur une évaluation du niveau de HbA1c, de HbC1c et de HbS1c glycosylées, ledit procédé comprenant une étape consistant à utiliser un anticorps selon la revendication 1 ou 2.

10. Procédé selon la revendication 9, dans lequel le prélèvement comprend une HbA, une HbS et/ou une HbC glycosylées hétérozygotes.

11. Procédé selon la revendication 9, où ledit procédé est destiné à déterminer le niveau de HbS et/ou de HbC glycosylées.
